(19) Europäisches Patentamt
European Patent Office
Office européen des brevets

(11) Publication number: **0 176 049**
**B1**

(12) # EUROPEAN PATENT SPECIFICATION

(45) Date of publication of patent specification: **13.06.90**

(21) Application number: **85111923.0**

(22) Date of filing: **20.09.85**

(51) Int. Cl.[5]: **C 07 C 215/30,** C 07 C 217/72, C 07 D 295/08, A 61 K 31/135

(54) **Aminoalkylnaphtalene derivatives having pharmacological activities.**

(30) Priority: **28.09.84 IT 2289784**

(43) Date of publication of application: **02.04.86 Bulletin 86/14**

(45) Publication of the grant of the patent: **13.06.90 Bulletin 90/24**

(84) Designated Contracting States: **AT BE CH DE FR GB IT LI LU NL SE**

(56) References cited:
**CH-A- 415 602**
**FR-A-2 288 518**
**US-A-2 629 738**

**BULLETIN DE LA SOCIETE CHIMIQUE DE FRANCE, no. 11-12, November-December 1975, pages 2759-2762; M. GUETTE et al.: "Obtention d'aminohydroxyesters par action du réactif de Réformatsky sur les bases de Mannich"**

(73) Proprietor: **CONSIGLIO NAZIONALE DELLE RICERCHE**
**Piazzale Aldo Moro, 7**
**I-00185 Roma (IT)**

(73) Proprietor: **MEDIOLANUM FARMACEUTICI s.r.l.**
**Via S.G. Cottolengo, 31**
**I-20143 Milano (IT)**

(72) Inventor: **Paroli, Eugenio, c/o UNIVERSITA' DI MILANO**
**ISTITUTO DI CHIMICA INDUSTRIALE**
**Via Golgi, 19, I-20133 Milan (IT)**
Inventor: **Nencini, Paolo, c/o UNIVERSITA' DI MILANO**
**ISTITUTO DI CHIMICA INDUSTRIALE**
**Via Golgi, 19, I-20133 Milan (IT)**
Inventor: **Anania, Maria Cristina, c/o UNIVERSITA' DI MILANO**
**ISTITUTO DI CHIMICA INDUSTRIALE**
**Via Golgi, 19, I-20133 Milan (IT)**
Inventor: **Maiorana, Stefano, c/o UNIVERSITA' DI MILANO**
**ISTITUTO DI CHIMICA INDUSTRIALE**
**Via Golgi, 19, I-20133 Milan (IT)**
Inventor: **Alemagna, Andreina, c/o UNIVERSITA' DI MILANO**
**ISTITUTO DI CHIMICA INDUSTRIALE**
**Via Golgi, 19, I-20133 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Licandro, Emanuela, c/o UNIVERSITA' DI MILANO**
**ISTITUTO DI CHIMICA INDUSTRIALE**
**Via Golgi, 19, I-20133 Milan (IT)**
Inventor: **Mainoli, Luigia, c/o UNIVERSITA' DI MILANO**
**ISTITUTO DI CHIMICA INDUSTRIALE**
**Via Golgi, 19, I-20133 Milan (IT)**

(74) Representative: **Bianchetti, Giuseppe**
**Studio Consulenza Brevettuale Via Rossini, 8**
**I-20122 Milan (IT)**

**Description**

The present invention concerns aminoalkylnaphtalene derivatives having formula I

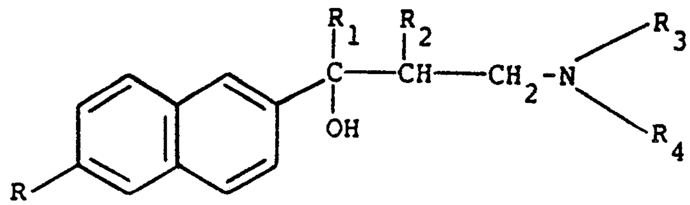

(I)

wherein:

R represents a hydroxy or $C_1$—$C_3$ alkoxy group;

$R_1$ represents a $C_1$—$C_5$ linear or branched alkyl group;

$R_2$ is hydrogen, methyl or ethyl;

$R_3$ and $R_4$, which are the same or different, represent hydrogen or $C_1$—$C_4$ alkyl groups, or, taken together with the nitrogen atom to which they are bound, form an optionally substituted pyrrolidine or piperidine group; enantiomer and diastereoisomers thereof; and salts thereof with pharmaceutically acceptable acids.

The term "$C_1$—$C_5$ linear or branched alkyl group" comprises, within the meaning thereof, methyl, ethyl, propyl, isopropyl, n-butyl, sec-butyl, ter-butyl, pentyl, isopentyl groups etc.

The compounds I, wherein $R_2$ is different from hydrogen, have two distinct asymmetric centers and they are therefore in form of diastereoisomeric pairs, which also comprised within the scope of the invention.

The invention concerns, of course, both the single optically active isomers, and the racemic mixtures thereof.

Preferred compounds according to the invention are those wherein R is an alkoxy group, namely a methoxy, propoxy or isopropoxy group, $R_1$ is methyl, ethyl, n-propyl, isopropyl or t-butyl, $R_2$ is methyl or ethyl, $R_3$ is methyl or t-butyl and $R_4$ is methyl or, when $R_3$ is t-butyl, hydrogen.

Even more preferred compounds are those wherein R is methoxy, $R_1$ is ethyl or t-butyl, $R_2$, $R_3$ and $R_4$ are methyl groups. Similar compounds are disclosed in FR—A—2288518, US—2629738, CH 415602 and in Bull. Soc. Chem. Fr. 11—12, Nov-Dec 1975, pages 2759—2762, as local anesthetic, antiphlogistic, analgesic, antipyretic, antiatherosclerotic or cardiovascular agents. No mention is made of possible central analgesia due to interaction with opioid receptors, which is a particular feature of the compounds of the invention.

2-(3-dimethylamino-1-propyl-2-methyl-1-hydroxypropyl)-6-isopropoxy-naphtalene;
2-(3-t-butylamino-1-propyl-2-methyl-1-hydroxypropyl)-6-isopropoxy-naphtalene;
2-(3-t-butylamino-1-isopropyl-2-methyl-1-hydroxypropyl)-6-isopropoxy-naphtalene;
2-(3-t-butylamino-1-isopropyl-2-methyl-1-hydroxypropyl)-6-methoxy-naphtalene;
2-(3-dimethylamino-1-isopropyl-2-methyl-1-hydroxypropyl)-6-methoxy-naphtalene;
2-(3-t-butylamino-1-isopropyl-2-methyl-1-hydroxypropyl)-6-propoxy-naphtalene;
2-(3-dimethylamino-1-isopropyl-2-methyl-1-hydroxypropyl)-6-propoxy-naphtalene;
2-(3-dimethylamino-1-isopropyl-2-methyl-1-hydroxypropyl)-6-isopropoxy-naphtalene;
2-(3-t-butylamino-1-isopropyl-2-methyl-1-hydroxypropyl)-6-isopropoxy-naphtalene;
2-(3-t-butylamino-1-ethyl-2-methyl-1-hydroxypropyl)-6-propoxy-naphtalene;
2-(3-dimethylamino-1-ethyl-2-methyl-1-hydroxypropyl)-6-propoxy-naphtalene;
2-(3-t-butylamino-1-ethyl-2-methyl-1-hydroxypropyl)-6-isopropoxy-naphtalene;
2-(3-dimethylamino-1-ethyl-2-methyl-1-hydroxypropyl)-6-isopropoxy-naphtalene;
2-(3-diethylamino-1-ethyl-2-hydroxypropyl)-6-methoxynaphtalene;
2-(3-dimethylamino-1-t-butyl-1-hydroxypropyl)-6-methoxynaphtalene;
2-(3-isopropylamino-1-ethyl-2-methyl-1-hydroxypropyl)-6-hydroxy-naphtalene.

The compounds I and isomers and diastereoisomers thereof may be salified with non toxic pharmaceutically acceptable organic or inorganic acids such as, for instance, hydrochloric, hydrobromic, sulfuric, methansulphonic, formic, acetic, oxalic, malonic, succinic, tartaric, malic, fumaric, citric, lactic, glutamic, aspartic acid, etc.

Further objects of the invention are provided by a process for the preparation of compounds I and by pharmaceutical compositions containing them as the active principle.

The compounds I are endowed with a series of pharmacological properties which allow different therapeutic applications in human medicine.

Namely, the compounds of the invention proved to be endowed, at different extents, with analgesic-narcotic properties, $H_1$- and $H_2$-antihistaminic, antisecretory, antiulcer, antiarrhythmic and anticholinergic activities. The compunds I turned out to be also able to inhibit the abstinence syndrome sustained by morphine and, generally, by other opioid alkaloids.

Finally, interactions with important enzymatic system, such as those controlling the prostaglandins biosynthesis or the enkephalins degradation, have also been noticed.

The compounds of the invention or salts thereof may be therefore administered by the oral or parenteral route, in form of pharmaceutical compositions, in admixture with suitable carriers and excipients.

One of the most important therapeutic indications is the treatment of drug addiction conditions, problem of increasing seriousness and diffusion.

The compounds I are prepared according to the invention starting from naphtalenes of formula II

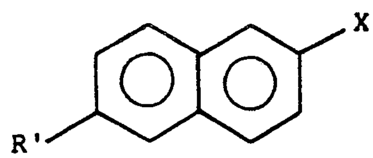

(II)

wherein R′ is a $C_1$—$C_3$ alkoxy group, or a suitably protected hydroxy group, and X is a chlorine, bromine or iodine atom, which, after treatment with strong bases such as lithium alkyl, sodium or potassium amides, lithium diisopropylamide etc., are reacted with aminoketones of formula III

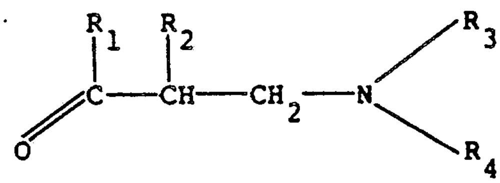

(III)

wherein $R_1$, $R_2$, $R_3$ and $R_4$ have the above defined meanings.

The reaction between the compounds II and the strong bases are normally carried out with a slight stoichiometric excess of the latter in anhydrous solvents such as tetrahydrofuran, ethyl ether, petroleum ether etc., at temperatures ranging from −30 to −50°C. The reaction with compounds III is carried out at the same temperature and in the same solvent.

The compounds of formula I wherein R = OH may be finally obtained from the corresponding alkoxy derivatives, for instance by treatment with 48% HBr, or starting from a compound II having $R_1$ representing a suitably protected hydroxy group. Examples of suitable protecting group comprise benzyl, tetrahydropyranyl, trityl, benzyloxycarbonyl, trifluoroacetyl group, etc. The protecting group cleavage is carried out by known methods, depending on the used group.

The compounds I so obtained may then be optionally subjected to salification, separation of the diastereoisomeric pairs, optical resolution of the enantiomers, using conventional methods.

The following examples further illustrate the invention without limiting in any way the scope thereof.

## Example 1

0.1 M (27.3 g) of 2-bromo-6-methoxynaphtalene were dissolved in 180 ml of anhydrous tetrahydrofuran; at −50°, 0.135 m of butyl lithium (corresponding to 85 ml of 1.6 M solution in hexane) were added. After 10 minutes 0.1 m (14.1 g) of 1-(dimethylamino)-2-methyl-pentan-3-one, dissolved in 35 ml of anhydrous tetrahydrofuran, were added dropwise at room temperature. The reaction mixture was then added with 180 ml of ethyl ether and acidified with 280 ml of a 10% hydrochloric acid solution. The aqueous phase was separated, alkalinized with 36% NaOH and extracted with ether. The organic phase, separated and dried on sodium sulphate, was evaporated under vacuum. The oily residue spontaneously solidifies after several days: it was then washed with petroleum ether and crystallized from hexane.

Yield 56.95%. M.p. 87—89°C.

Elemental analysis:

| | C | H | N |
|---|---|---|---|
| found % | 75.92 | 8.96 | 4.74 |
| calc. % (for $C_{19}H_{27}NO_2$) | 75.75 | 8.97 | 4.65. |

## Example 2

0.1 M (30.7 g) of 2-bromo-6-hydroxy-naphtalene dihydropyranylether were dissolved in 120 ml of anhydrous tetrahydrofuran; 0.12 m of butyl lithium (corresponding to 77 ml of a 1.6M solution in hexane) were added at −50°C. After 30 minutes, 0.1 m (14.1 g) of 1-(dimethylamino)-2-methyl-pentan-3-one in 30 ml of anhydrous tetrahydrofuran were added dropwise. After 30 hours the mixture was allowed to warm to

room temperature and after 3 more hours the mixture was acidified with 250 ml of a 10% hydrochloric acid solution (cleaving thereby the dihydropyranylether group). After 1 hour the product formed was filtered and crystallized from a 10:1 methanol:isopropanol mixture.

Elemental analysis:

| | C | H | N |
|---|---|---|---|
| found % | 66.98 | 8.08 | 4.45 |
| calc. % (for $C_{18}H_{26}ClNO_2$) | 66.76 | 8.03 | 4.32. |

Example 3

0.1 M (23.7 g) of 2-bromo-6-methoxynaphtalene were dissolved in 180 ml of anhydrous tetrahydrofuran; 0.135 m of butyl lithium (corresponding to 85 ml of 1.6 M solution in hexane) were added thereto. After 10 minutes, 0.1 m (14.1 g) of 1-(dimethylamino)-4,4-dimethyl-pentan-3-one in 35 ml of anhydrous tetrahydrofuran, keeping the temperature between −50° and −30°C, were added dropwise. After 2 hours, the temperature was allowed to raise to room temperature; the reaction mixture was diluted with 180 ml of ethyl ether and acidified with 280 ml of a 10% hydrochloric acid; the aqueous phase was separated, alkalinized with conc. NaOH, extracted with ethyl ether and dried on $Na_2SO_4$; the solvent was evaporated under vacuum and the oily residue was chromatographed on silica gel; eluent ethyl acetate. The obtained product was washed with hexane. M.p. 98—100°C. Yield 41.35%.

Elemental analysis:

| | C | H | N |
|---|---|---|---|
| found % | 76.50 | 9.44 | 4.51 |
| calc. % (for $C_{19}H_{29}NO_2$) | 76.19 | 9.21 | 4.44. |

PHARMACOLOGICAL TESTING

The compounds of the previous examples have been pharmacologically tested.

The compound of Example 2 turned out to be endowed with analgesic activity, with an $ED_{50}$ in the hot plate test of 5.6 ± 1.51 mg/kg/i.p. in the mouse and with anti-inflammatory activity in the paw oedema by carrageenin according to Randall-Selitto, with an $ED_{50}$ of 1.25 mg/kg, always i.p., in the rat.

Also the compound of Example 1 proved to be active in the hot plate test, with an $ED_{50}$ of 13.5 mg/kg and with a kind of activity partially reversible by naloxone. The compounds of Examples 1 and 3 finally proved to be active in inhibiting the enzymes responsible for the enkephalins hydrolysis with $I.C._{50}$ of 4.0 and 5.0 mM, respectively.

Finally, the acute toxicity has been studied in the mouse, by i.p. administration: the $LD_{50}$ values are reported in the following table.

TABLE
ACUTE TOXICITY IN THE MOUSE

| Compound ex. | $LD_{50}$ (mg/kg i.p.) |
|---|---|
| 1 | 65,8481 ± 2,659 |
| 2 | 56,16 ± 4,85 |
| 3 | 120,96 ± 5,32. |

The present invention refers also to all the industrially applicable aspects connected with the use of compounds I as therapeutic agent. An essential aspect of the invention is therefore provided by pharmaceutical compositions containing predetermined and therapeutically effective amounts of at least one of compounds I or of salts thereof, in admixture with excipients conventionally used in pharmaceutical technique and optionally in combination with other active principles. Examples of said pharmaceutical comprise tablets, sugar coated tablets, capsules, syrups, sachets, sterile solutions for intramuscular or intravenous injections, suppositories etc.

**Claims for the Contracting States: BE CH DE FR GB IT LI LU NL SE**

1. Compounds of formula I

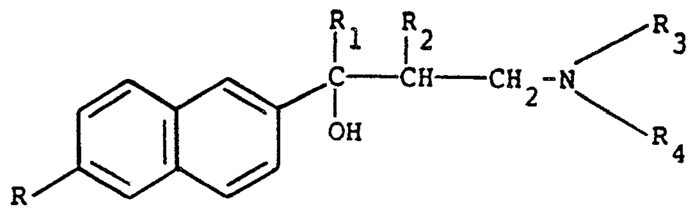

(I)

wherein:

R represents a hydroxy or $C_1$—$C_3$ alkoxy group;

$R_1$ represents a $C_1$—$C_5$ linear or branched alkyl group;

$R_2$ is hydrogen, methyl or ethyl;

$R_3$ and $R_4$, which are the same or different, represent hydrogen or $C_1$—$C_4$ alkyl groups; enantiomer and diastereoisomers thereof; and salts thereof with pharmaceutically acceptable acids.

2. Compounds according to claim 1 wherein $R_1$ is ethyl, $R_2$, $R_3$ and $R_4$ are methyl.

3. Compounds according to claim 1 wherein $R_1$ is t-butyl, $R_2$ is hydrogen, $R_3$ and $R_4$ are methyl.

4. A process for the preparation of compounds of formula I characterized in that compounds of formula II

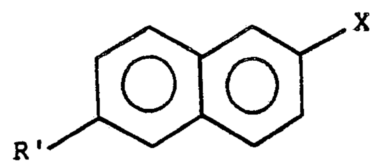

(II)

wherein R′ is a $C_1$—$C_3$ alkoxy group or a suitably protected hydroxy group, and X is a chlorine, bromine or iodine atom, are reacted with lithium-alkyls in anhydrous aprotic solvents and then with aminoketones having formula III

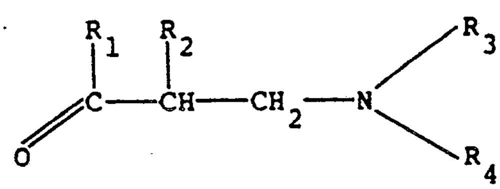

(III)

wherein $R_1$, $R_2$ and $R_3$ and $R_4$ have the above defined meanings.

5. A process according to claim 4 characterized by using butyl-lithium in anhydrous tetrahydrofuran as a solvent, at temperatures ranging from −30 to −50°C.

6. Pharmaceutical compositions containing as the active principle at least one of the compounds of claims 1—3 in admixture with pharmaceutically acceptable excipients.

7. Pharmaceutical compositions according to claim 6 in form of tablets, capsules, syrups, granules, suspensions, solutions, suppositories, vials, bottles.

**Claims for the Contracting State: AT**

1. Process for the preparation of compounds having formula I

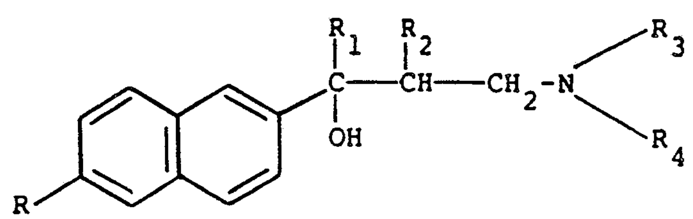

(I)

wherein:

R represents a hydroxy or $C_1$—$C_3$ alkoxy group;

$R_1$ represents a $C_1$—$C_5$ linear or branched alkyl group;

$R_2$ is hydrogen, methyl or ethyl;

$R_3$ and $R_4$, which are the same or different, represent hydrogen or $C_1$—$C_4$ alkyl groups; enantiomer and diastereoisomers thereof; and salts thereof with pharmaceutically acceptable acids, characterized in that compounds of formula II

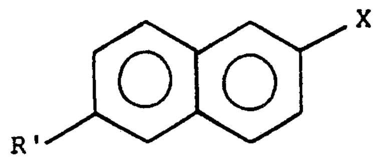

(II)

wherein R′ is a $C_1$—$C_3$ alkoxy group or a suitably protected hydroxy group, and X is a chlorine, bromine or iodine atom, are reacted with lithium-alkyls in anhydrous aprotic solvents and then with aminoketones having formula III

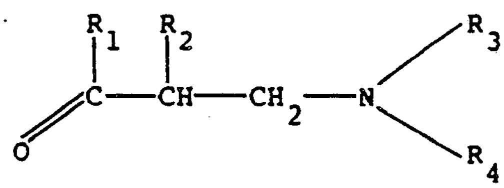

(III)

wherein $R_1$, $R_2$ and $R_3$ and $R_4$ have the above defined meanings.

2. A process according to claim 4 characterized by using butyl lithium in anhydrous tetrahydrofuran as a solvent, at temperatures ranging from −30 to −50°C.

**Patentansprüche für die Vertragsstaaten: BE CH DE FR GB IT LI LU NL SE**

1. Verbindungen der Formel I

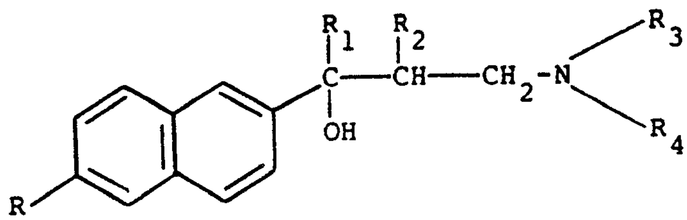

(I)

wobei:

R eine Hydroxy- oder $C_1$—$C_3$ Alkoxygruppe ist;

$R_1$ ein $C_1$—$C_5$ lineare oder verzweigte Alkylgruppe ist;

$R_2$ Wasserstoff, Methyl oder Äthyl ist;

$R_3$ und $R_4$, die gleich oder verschieden sein können, Wasserstoff oder $C_1$—$C_4$ Alkylgruppen sind; Enantiomere und Diastereoisomere davon; und deren Salz mit pharmazeutisch zulässigen Säuren.

2. Verbindungen nach Anspruch 1, wobei $R_1$ Äthyl, $R_2$, $R_3$ und $R_4$ Methyl sind.

3. Verbindungen nach Anspruch 1, wobei $R_1$ t-Butyl, $R_2$ Wasserstoff, $R_3$ und $R_4$ Methyl sind.

4. Verfahren zur Herstellung von Verbindungen der Formel I, dadurch gekennzeichnet, daß Verbindungen der Formel II

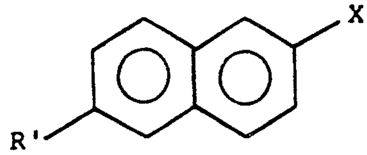

(II)

wobei R′ eine $C_1$—$C_3$-Alkoxygruppe oder eine geeignet geschützte Hydroxylgruppe ist, und X ein Chlor-, Brom- oder Jodatom ist, mit Lithium-Alkylen in nichtwässrigen aprotischen Lösungsmitteln und dann mit Aminoketonen der Formel III

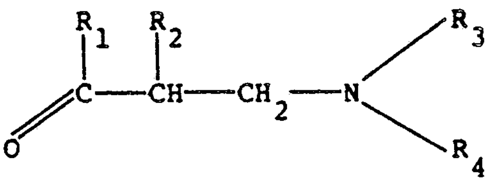

(III)

zur Reaktion gebracht werden, wobei $R_1$, $R_2$ und $R_3$ und $R_4$ die o.a. Bedeutungen besitzen.

5. Verfahren nach Anspruch 4, dadurch gekennzeichnet, dass als Lithium-Alkyl Butyl-Lithium in nichtwässrigem Tetrahydrofuran als ein Lösungsmittel, in einem Temperaturbereich von −30 bis −50°C, verwendet wird.

6. Pharmazeutische Zusammensetzungen, die als aktiven Bestandteil wenigstens eine der Verbindungen der Ansprüche 1—3 in einer Mischung mit pharmazeutisch zulässigen Exzipienten enthalten.

7. Pharmazeutische Zusammensetzungen nach Anspruch 6 in Form von Tabletten, Kapseln, Sirupen, Granuli, Suspensionen, Lösungen, Suppositorien, Ampullen, Flaschen.

**Patentansprüche für den Vertragsstaat: AT**

1. Verfahren zur Herstellung von Verbindungen der Formel I,

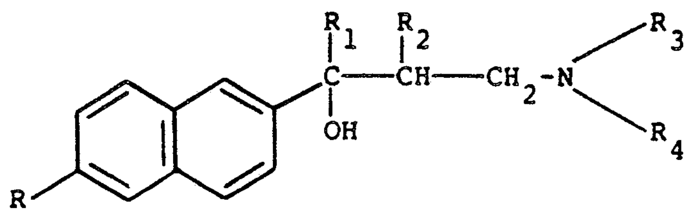

(I)

wobei:

R eine Hydroxy- oder $C_1$—$C_3$ Alkoxygruppe ist;

$R_1$ ein $C_1$—$C_5$ lineare oder verzweigte Alkylgruppe ist;

$R_2$ Wasserstoff, Methyl oder Äthyl ist;

$R_3$ und $R_4$, die gleich oder verschieden sein können, Wasserstoff oder $C_1$—$C_4$ Alkylgruppen sind; Enantiomere und Diastereoisomere davon; und deren Salz mit pharmazeutisch zulässigen Säuren, dadurch gekennzeichnet, daß Verbindungen der Formel II

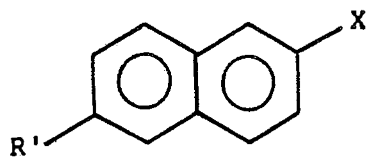

(II)

wobei R′ eine $C_1$—$C_3$-Alkoxygruppe oder eine geeignet geschützte Hydroxylgruppe ist, und X ein Chlor-, Brom- oder Jodatom ist, mit Lithium-Alkylen in nichtwässrigen aprotischen Lösungsmitteln und dann mit Aminoketonen der Formel III

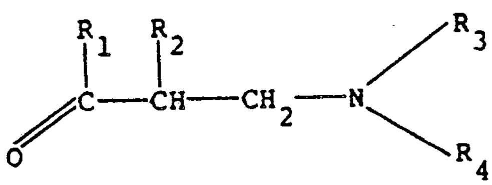

(III)

zur Reaktion gebracht werden, wobei $R_1$, $R_2$ und $R_3$ und $R_3$ die o.a. Bedeutungen besitzen.

2. Verfahren nach Anspruch 1, dadurch gekennzeichnet, dass als Lithium-Alkyl Butyl-Lithium in nichtwässrigem Tetrahydrofuran als ein Lösungsmittel, in einem Temperaturbereich von −30 bis −50°C, verwendet wird.

**Revendications pour les Etats contractants: BE CH DE FR GB IT LI LU NL SE**

1. Composés de formule I

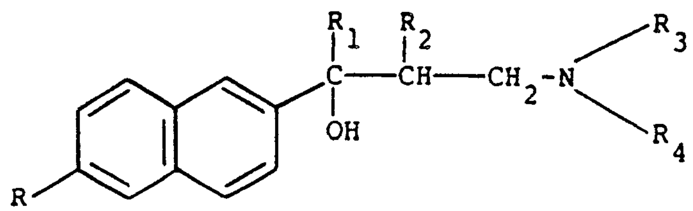

(I)

dans laquelle:

R représente un groupe hydroxy ou alcoxy en $C_1$—$C_3$;

$R_1$ représente un groupe alcoyle linéaire ou ramifié en $C_1$—$C_5$;

$R_2$ est l'hydrogène, le radical méthyle ou le radical éthyle;

$R_3$ and $R_4$, qui sont identiques ou différents, représentent l'hydrogène ou des groupes alcoyle en $C_1$—$C_4$; leur énantiomères et diastérèo-isomères; et leurs sels avec des acides pharmaceutiquement acceptables.

2. Composés selon la revendication 1, dans lesquels $R_1$ représente le radical éthyle, $R_2$, $R_3$ et $R_4$ représentent le radical méthyle.

3. Composés selon la revendication 1, dans lesquels $R_1$ représente le radical t-butyle, $R_2$ représente l'hydrogène, $R_3$ et $R_4$ représentent le radical méthyle.

4. Procédé pour la préparation des composés de formule I caractérisé en ce que des composés de formule II

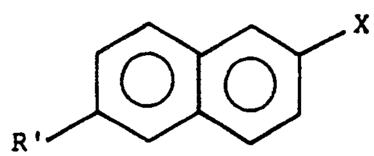

(II)

dans laquelle R' représente un groupe alcoxy en $C_1$—$C_3$ ou un groupe hydroxy protégé de façon approprié, et X représente un atome de chlore, de brome, ou d'iode, sont mis en réaction avec des alcoyles-lithium dans des solvants aprotiques anhydres, puis avec des aminocétones ayant la formule III

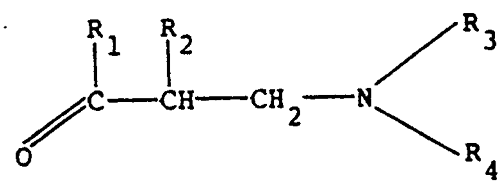

(III)

dans laquelle $R_1$, $R_2$ et $R_3$ et $R_4$ possèdent les significations définies ci-dessus.

5. Procédé selon la revendication 4, caractérisé par l'utilization de butyl-lithium dans du tétrahydrofuranne anhydre à des températures allant de −30 à −50°C.

6. Compositions pharmaceutiques contenant en tant que principe actif au moins l'un des composés des revendications 1—3 en mélange avec des excipients pharmaceutiquement acceptables.

7. Compositions pharmaceutiques selon la revendication 6 sous forme de comprimés, de capsules, de sirops, de granules, de suspensions, de solutions, de suppositoires, de flacons, de bouteilles.

**Revendications pour l'Etat contractant: AT**

1. Procédé pour la préparation des composés de formule I

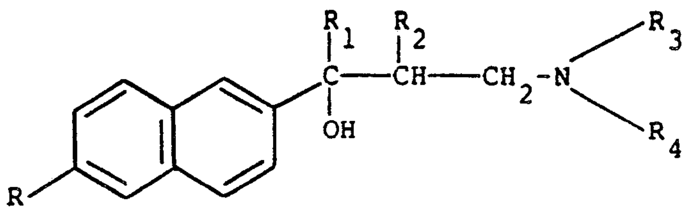

(I)

dans laquelle:

R représente un groupe hydroxy ou alcoxy en $C_1$—$C_3$;

$R_1$ représente un groupe alcoyle linéaire ou ramifié en $C_1$—$C_5$;

$R_2$ est l'hydrogène, le radical méthyle ou le radical éthyle;

$R_3$ and $R_4$, qui sont identiques ou différents, représentent l'hydrogène ou des groupes alcoyle en $C_1$—$C_4$; leur énantiomères et diastéréo-isomères; et leurs sels avec des acides pharmaceutiquement acceptables, caractérisé en ce que des composés de formule II

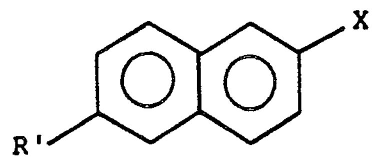

(II)

dans laquelle R' représente un groupe alcoxy en $C_1$—$C_3$ ou un groupe hydroxy protégé de façon approprié, et X représente un atome de chlore, de brome, ou d'iode, sont mis en réaction avec des alcoyles-lithium dans des solvants aprotiques anhydres, puis avec des aminocétones ayant la formule III

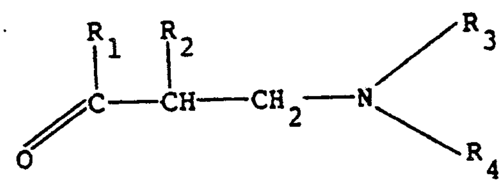

(III)

dans laquelle $R_1$, $R_2$ et $R_3$ et $R_4$ possèdent les significations définies ci-dessus.

2. Procédé selon la revendication 1, caractérisé par l'utilization de butyl-lithium dans du tétrahydrofuranne anhydre à des températures allant de −30 à −50°C.